**Europäisches Patentamt**

**European Patent Office**

**Office européen des brevets**

(19)

(11) Veröffentlichungsnummer: **0 175 807**
**B1**

(12) # EUROPÄISCHE PATENTSCHRIFT

(45) Veröffentlichungstag der Patentschrift:
**07.12.88**

(51) Int. Cl.⁴: **H 01 J 49/14**

(21) Anmeldenummer: **84111519.9**

(22) Anmeldetag: **27.09.84**

(54) Einrichtung zur Durchführung des SNMS-Verfahrens.

(43) Veröffentlichungstag der Anmeldung:
**02.04.86 Patentblatt 86/14**

(45) Bekanntmachung des Hinweises auf die Patenterteilung:
**07.12.88 Patentblatt 88/49**

(84) Benannte Vertragsstaaten:
**CH DE FR GB IT LI**

(56) Entgegenhaltungen:
**DE-A- 2 950 330**
**US-A- 3 660 655**
**US-A- 3 894 233**
**US-A- 3 930 155**

**APPLIED PHYSICS, Band 14, 20. September 1977,
Seiten 43-47, Springer-Verlag, Heidelberg, DE; H.
OECHSNER u.a.: "Sputtered neutral mass
spectrometry (SNMS) as a tool for chemical surface
analysis and depth profiling"
ZEITSCHRIFT FÜR NATURFORSCHUNG, Band 22A, Nr.
4, April 1967, Seiten 459-467, Tübingen, DE; H.E. BESKE:
"Untersuchungen zur Emission positiver
Sekundärionen aus festen Targets. Die Brauchbarkeit
der Ionenbeschuss-Ionenquelle in der
Massenspektroskopie"**

(73) Patentinhaber: **LEYBOLD AKTIENGESELLSCHAFT,
Bonner Strasse 498, D-5000 Köln 51 (DE)**

(72) Erfinder: **Meier, Stefan, Minze-Weg 38,
D-5000 Köln 51 (DE)**
Erfinder: **Müller, Karl-Heinz, Dr., Ludgeristrasse 21,
D-4712 Werne (DE)**

(74) Vertreter: **Leineweber, Jürgen, Dipl.-Phys.,
Nagelschmiedshütte 8, D-5000 Köln 40 (DE)**

## Beschreibung

Die Erfindung bezieht sich auf eine Einrichtung zur Durchführung des SNMS-Verfahrens mit einer separaten Ionenquelle, einem Probenträger, einer Vorrichtung zur Erzeugung eines HF-Plasmas und einem Massenanalysator.

Beim sogenannten SNMS-Verfahren (Sputtered oder Secondary Neutral Mass Spectrometry) erfolgt die Analyse einer Probe in der Weise, dass aus der Probenoberfläche durch Ionenbeschuss Neutralteilchen ausgelöst werden, diese in einem HF-Plasma ionisiert und danach massenspektrometrisch untersucht werden. Hierbei gibt es zwei Möglichkeiten, die Primärionen zu erzeugen.

Zu einem besteht die Möglichkeit, den Beschuss der Probe mit Ionen aus dem Plasma (Direct Bombardmet Mode / DBM) vorzunehmen. Dazu ist es erforderlich, die Probe im Bereich des Plasmas anzuordnen und zwischen dem HF-Plasma und der Probe die für den gewünschten Beschuss erforderliche Spannungsdifferenz anzulegen. Aus Appl. Phys. 14, 43–47 (1977) ist dieses DBM/SNMS-Verfahren bekannt. Dieses Verfahren kann bei leitenden und halbleitenden Proben angewandt werden. Bei Isolatoren kann man allerdings keinen Ionenbeschuss durch Anlegen einer Gleichspannung – wie dies in der zitierten Veröffentlichung beschrieben ist – hervorrufen.

Eine weitere Möglichkeit besteht darin, zum Beschuss der Probe eine separate Ionenquelle vorzusehen, um die Primärionenerzeugung und die Nachionisierung der Neutralteilchen durch das HF-Plasma zu entkoppeln. Diese SBM (Separate Bombardment Mode)-Betriebsart des SNMS-Verfahrens ist aus der DE-OS 29 50 330 bekannt. Bei der darin vorgeschriebenen Vorrichtung zur Durchführung dieses Verfahrens sind die separate Ionenquelle und die Probe auf der einen Seite des HF-Plasmas und der Massenanalysator auf der anderen Seite des Plasmas angeordnet. Zwischen der Probe und dem Hochfrequenzplasma befindet sich ein aus mehreren Blenden bestehendes Diaphragma. Dieses hat zum einen die Aufgabe, das Auftreffen von Ionen und Elektronen des Plasmas auf die Probe zu verhindern. Zum anderen soll es den Durchtritt von unmittelbar durch den Ionenbeschuss aus der Probe ausgelösten Sekundärionen verhindern, da diese beim SNMS-Verfahren unerwünscht sind und einen störenden Untergrund bilden können. Mit dieser vorbekannten Einrichtung können Isolatoren nur dann untersucht werden, wenn durch geeignete Massnahmen die durch den Ionenbeschuss hervorgerufenen Aufladungen kompensiert werden.

Nachteilig an den vorbekannten Einrichtungen ist, dass sie für vergleichende Messungen, einmal nach dem DBM/SNMS-Verfahren, einmal nach dem SBM/SNMS-Verfahren, nicht geeignet sind. Beim Übergang von einer SNMS-Betriebsart zur anderen sind entweder aufwendige Umbauten der Apparatur oder verschiedene Apparaturen erforderlich.

Der vorliegenden Erfindung liegt die Aufgabe zugrunde, eine Einrichtung der eingangs genannten Art zu schaffen, mit der eine Probe ohne apparativen Umbau sowohl nach dem DBM/SNMS-Verfahren als auch nach dem SBM/SNMS-Verfahren unmittelbar nacheinander untersucht werden kann.

Erfindungsgemäss wird diese Aufgabe dadurch gelöst, dass die Ionenquelle und der Massenanalysator nebeneinander auf derselben Seite des für das Hochfrequenzplasma vorgesehenen Raumes angeordnet sind und dass sich die auf dem Probenträger gehalterte Probe innerhalb des für das Hochfrequenzplasma vorgesehenen Raumes befindet. Bei der Durchführung des DBM/SNMS-Verfahrens bleibt die Ionenquelle abgeschaltet.

Zwischen dem HF-Plasma – vorzugsweise ein Argon-Plasma – und der Probe bzw. ihrer Probenhalterung wird die für den Beschuss der Probe mit Ionen aus dem Plasma erforderliche Spannung angelegt. Dies kann im Falle von Leitern und Halbleitern eine Gleichspannung sein. Im Falle von Isolatoren wird eine hochfrequente Wechselspannung an die Probenunterlage gelegt. Aufgrund der dielektrischen Eigenschaften des Isolators und aufgrund der unterschiedlichen Beweglichkeit der Ionen und Elektronen im Plasma liegt an der Oberfläche des Isolators ein negatives Gleichspannungspotential, was wiederum zur Beschleunigung der Ionen in Richtung Probe und damit zur Zerstäubung der Probe führt. Aus der Probe werden dadurch bei Leitern, Halb- und Nichtleitern Neutralteilchen ausgelöst. In Richtung Massenanalysator fliegende Neutralteilchen werden im Plasma ionisiert und im Massenanalysator auf ihre Masse hin untersucht. Zur Umschaltung vom DBM/SNMS-Betrieb auf SBM/SNMS-Betrieb ist es lediglich erforderlich, die Spannungsdifferenz zwischen Plasma und Probe so weit zu reduzieren, dass Ionen des Plasmas die Probe nicht mehr anregen bzw. zerstäuben, und den Beschuss der Probe mit der Ionenquelle vorzunehmen. Die mittlere freie Weglänge der Plasma-Teilchen ist so gross, dass der Primärionenstrom weitestgehend ungestört das Plasma durchsetzen kann. Durch den Ionenbeschuss ausgelöste Neutralteilchen werden vom Plasma ionisiert und im Massenanalysator untersucht.

Die Erfindung beruht im wesentlichen auf der Erkenntnis, dass es beim SBM/SNMS-Betrieb nicht erforderlich ist, mit Hilfe eines elektrischen Diaphragmas eine übermässige Belastung der Probe durch Plasmateilchen zu verhindern. Bereits eine geeignete Einstellung des Plasmapotentials reicht dazu aus. Auch die Unterdrückung von Sekundärionen durch ein Diaphragma ist nicht erforderlich, da meistens die Anzahl der emittierten Neutralteilchen erheblich grösser ist als die Anzahl der emittierten Sekundärionen. Ausserdem haben die unmittelbar ausgelösten Sekundärionen und die nachträglich ionisierten Neutralteilchen unterschiedliche Energieverteilungen, so dass die Sekundärionen mit Hilfe einer dem Massenanalysator vorgelagerten Ionenoptik (Set-

zen eines Energiefensters) unterdrückt werden können.

Aufgrund der günstigen Anordnung von Ionenquelle, Nachweissysem und Probe zueinander wird grosser – verglichen mit der vorbekannten Anordnung – Raumwinkel der zerstäubten Neutralteilchen vom Nachweissystem erfasst. Dies führt zu einer hohen Nachweisempfindlichkeit. Dadurch wiederum kann bei der vorgeschlagenen Vorrichtung eine Ionenkanone mit geringem Primärionenstrom eingesetzt werden, wie z.B. eine Feinfokusionenquelle, die zur Mikroanalyse eingesetzt werden kann. SNMS-Mikroanalysen mit einer lateralen Auflösung von 1μm sind möglich. Dies gilt für Metalle, Halb- und Nichtleiter. Bei Nichtleitern wird die beim Ionenbeschuss der Probe erzeugte Aufladung durch die Elektronenkomponente des Plasmas kompensiert, da die Probe direkten Kontakt zum Plasma hat.

Ein weiterer entscheidender Vorteil der erfindungsgemässen Vorrichtung liegt darin, dass diese ohne besondere Umbauten auch für die Durchführung des SIMS-Verfahrens geeignet ist. Beim SIMS-Verfahren (Secondary Ion Mass Spectrometry) wird eine Probe mit Ionen beschossen. Die durch diesen Ionenbeschuss unmittelbar ausgelösten Sekundärionen werden auf ihre Masse hin untersucht. SIMS könnte im Prinzip auch mit der aus der DE-OS 29 50 330 vorbekannten Einrichtung ausgeführt werden. Im Vergleich zu einer «idealen» SIMS-Konfiguration wäre das aber nur mit grossen Empfindlichkeitsverlusten (mehrere Grössenordnungen) möglich. Ein senkrechter Beschuss der Probe bei gleichzeitiger Analyse – wie dies für eine grosse Dynamik und gute Tiefenauflösung bei Tiefenprofilen erforderlich ist – kann ebenfalls mit der vorgeschriebenen Einrichtung nicht realisiert werden. Letzteres gilt nicht nur für SIMS-, sondern auch für SNMS-Analysen. Schliesslich können Mikroanalysen aus Intensitätsgründen ebenfalls nicht durchgeführt werden.

Zur Durchführung des SIMS-Verfahrens mit der erfindungsgemässen Einrichtung ist es lediglich erforderlich, das HF-Plasma abzuschalten. Die Probe kann danach unter optimalen räumlichen Bedingungen nach dem SIMS-Verfahren untersucht werden. In Analogie zum SNMS-Betrieb können auch SIMS-Mikroanalysen durchgeführt werden.

Bei ausgeschaltetem Plasma können durch Anregung mit einer Feinfokusionenquelle auch Sekundärelektronenbilder im Sub-μm-Bereich aufgenommen werden. Der hierzu erforderliche Detektor kann auf der Nachweisseite angebracht und beim Plasmabetrieb durch eine Blende vor Bestäubung geschützt werden.

Unmittelbare SIMS-SNMS-Vergleichsmessungen unter optimalen Bedingungen (d.h. mit grösster Nachweisempfindlichkeit für beide Verfahren) waren in der Vergangenheit nicht möglich. Sie erforderten bisher stets unterschiedliche Messgeräte oder zumindest umfangreiche Umbauten. SIMS-SNMS-Vergleichsmessungen sind jedoch häufig sehr erwünscht, da sich diese beiden Spektroskopier-Verfahren ergänzen. Mit dem SNMS-Verfahren sind quantitative Bestimmungen der Zusammensetzung der Probe möglich. Demgegenüber hängt beim SIMS-Verfahren die Entstehung von Sekundärionen an der Probenoberfläche auf komplizierte Weise von der Oberflächenbeschaffenheit ab, so dass Empfindlichkeitsschwankungen von mehreren Zehnerpotenzen auftreten können. Mit dem SIMS-Verfahren sind deshalb häufig hochempfindliche, aber nicht quantitative Analysen möglich. Sekundärionen-Spektren können aufgrund der erwähnten Matrixabhängigkeit der Sekundärionen-Emission chemische Informationen (z.B. über Abbindungen und Bindungszustände) liefern.

Die unmittelbare Durchführung vergleichender SIMS-SNMS-Messungen ist mit der erfindungsgemässen Einrichtung deshalb möglich, weil die Probe in eine Position gebracht werden kann, die sowohl für SIMS als auch für beide SNMS-Betriebsarten optimal günstig ist. Das SBM/SNMS-Verfahren kann gegenüber dem durch die DE-OS 29 50 330 gegebenen Stand der Technik mit höherer Nachweisempfindlichkeit durchgeführt werden, da das den Raumwinkel begrenzende Diaphragma entfallen ist. Darüber hinaus kann der Abstand zwischen Probe und Nachweissystem im Vergleich zur vorveröffentlichten Anordnung mindestens halbiert werden. Dadurch wird zwar beim DBM die Nachionisierungswahrscheinlichkeit auch etwa halbiert. Aber die Anzahl der auf die Öffnung der Ionenoptik treffenden zerstäubten Teilchen ist etwa viermal grösser. Dies gilt sogar ohne Berücksichtigung der Raumwinkelbegrenzung durch das elektrische Diaphragma. Insgesamt ergibt sich für alle erwähnten Messverfahren die günstigste geometrische Anordnung zur Erzielung maximaler Leistungsdaten. Ein schneller Wechsel zwischen SIMS und SNMS sowie zwischen den beiden SNMS-Betriebsarten ist durch einfaches Umschalten möglich geworden.

Weitere Vorteile und Einzelheiten der Erfindung sollen anhand von in den Figuren 1 bis 3 dargestellten Ausführungsbeispielen erläutert werden. Es zeigen:

Fig. 1 eine kombinierte SIMS-SNMS-Apparatur,

Fig. 2 vergrössert die Probenaufnahme von Fig. 1 und

Fig. 3 eine weitere Probenaufnahme.

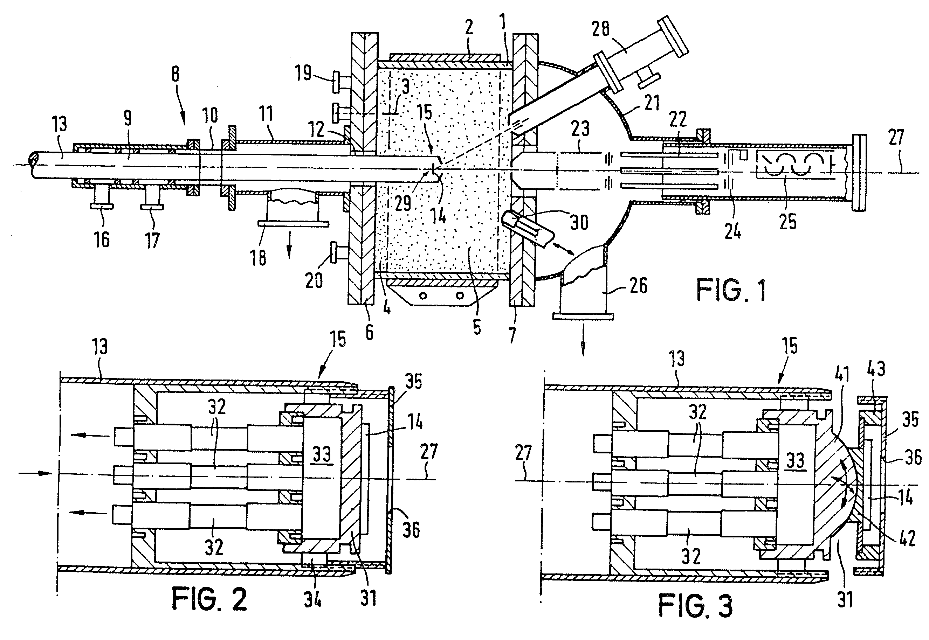

Die in Fig. 1 dargestellte Apparatur umfasst zentral das Plasmagefäss 1, das von einer einwindigen, aus einem Blechstreifen bestehenden HF-Spule 2 umgeben ist. Mittels einer Sonde 3 erfolgt die Kontrolle des von der Spule 2 im Innenraum 4 erzeugten, durch Punktraster angedeuteten Plasmas 5.

Das Plasmagefäss 1 ist mit seitlichen Flanschen 6 und 7 ausgerüstet. An den Flansch 6 ist eine Probenschleuse 8 gehaltert. Diese besteht aus dem Schleusengrundkörper 9, dem Ventil 10 und einer Zwischenkammer 11, die über die Öffnung 12 im Flansch 6 mit dem Innenraum 4 des Plasmagefässes 1 in Verbindung steht. Durch den Schleusengrundkörper 9, die Zwischenkammer 11 und die Öffnung 12 im Flansch 6 erstreckt sich der in Messposition dargestellte Probenstab 13, auf dem endständig die Probe 14 gehalten ist. Die insgesamt mit 15 bezeichnete Probenaufnahme ist in Fig. 2 vergrössert dargestellt. Die Anschlussstutzen 16 und 17 am Schleusengrundkörper 9 sowie 18 an der Zwischenkammer 11 dienen dem Anschluss von Vakuumpumpen. Weitere Anschlussstutzen 19 und 20 am Flansch 6 dienen der Druckmessung bzw. dem Einlass von Arbeitsgas, vorzugsweise Argon, für das Plasma 5.

Auf der gegenüberliegenden Seite des Plasmagefässes 1 ist am Flansch 7 ein Gehäuse 21 für ein Massenspektrometer befestigt. Dieses umfasst ein Quadrupol-Massenfilter 22, dem eine den Flansch 7 durchsetzende und mit ihrer Eintrittsöffnung bis zum Plasma 5 reichenden Ionenoptik 23 für das Setzen eines Energiefensters vorgelagert ist. Dem Quadrupol 22 nachgeordnet ist eine weitere Ionenoptik 24, welche die zu registrierenden Ionen auf einen axial versetzt angeordneten Multiplier 25 ablenkt. Das Gehäuse 21 ist mit dem Stutzen 26 zum Anschluss einer Hochvakuumpumpe ausgerüstet. Die Evakuierung des Innenraumes 4 des Plasmagefässes 1 erfolgt durch die an die Stutzen 18 und 26 angeschlossenen Vakuumpumpen.

Schleuse 8, Plasmagefäss 1, Ionenoptik 23 und Quadrupol 22 sind gleichachsig angeordnet. Die Achse des Gesamtsystems ist mit 27 bezeichnet.

Am Flansch 7 ist neben dem Massenspektrometer eine Ionenkanone 28 gehalten. Diese ist so angeordnet, dass ihr Fokuspunkt auf die Probe 14 in ihrer dargestellten Messposition gerichtet ist. Die Probe kann kippbar (Pfeil 29) gehalten sein, so dass sie senkrecht zur Richtung der auftreffenden Ionen angeordnet werden kann.

Weiterhin ist am Flansch 7 ein Sekundärelektronen-Detektor 30 gehalten. Er kann verschiebbar ausgeführt sein, so dass er sich nur im Falle der Aufnahme von Sekundärelektronenbildern in seiner Messposition befindet. Beim Plasmabetrieb wird er zurückgezogen und mittels einer nicht dargestellten Blende vor Bestäubung geschützt.

Fig. 2 zeigt die Ausbildung des probenseitigen Endes des Probenstabes 13. Dort befindet sich ein hohler Probenträger 31, auf dem die Probe 14 durch eine nicht dargestellte Klemme befestigt ist. Über eine Zu- und zwei Abführungsleitungen 32 erfolgt eine Kühlmittelversorgung des Innenraumes 33 des Probenträgers 31. Die Leitungen 32 sowie die Halterungen 34 des Probenträgers 31 bestehen aus elektrisch isolierendem Material, so dass der Probenträger 31 und damit die Probe 14 auf ein bestimmtes Potential gelegt werden kann.

Der Probe 14 ist noch eine in axialer Richtung verstellbare Kappe 35 mit einer Beschussöffnung 36 zugeordnet. Diese verschiebbare Kappe hat die Aufgabe, einen homogenen Probenabtrag zu ermöglichen (Appl. Phys. 20 (1979), S. 55–60).

Die Probenoberfläche kann gekippt werden (z.B. senkrecht zum einfallenden Ionenstrahl oder parallel zur Öffnung der Ionenoptik). Ein Ausführungsbeispiel dafür zeigt Fig. 3. Der Probenträger 31 umfasst dazu eine Kalotte 41 mit einem entsprechend geformten, darauf gleitenden Bauteil 42, auf dem die Probe 14 gehalten ist. Über einen Keramikring 43 trägt das Bauteil 42 ausserdem noch die Kappe 35.

Um mit der in Fig. 1 dargestellten Apparatur eine Probe 14 nach dem DBM/SNMS-Verfahren zu untersuchen, wird die einwindige Spule 2 in bekannter Weise derart mit Strom versorgt, dass das Plasma 5 entsteht. Die Ionenkanone 28 ist abgeschaltet. Die Probe 14 bzw. ihr Probenträger 31 werden auf ein solches Potential (z.B. 0 bis einige kV) gelegt, dass die Ionen des Plasmas 5 auf die Probe 14 auftreffen. Beim DBM wird im Falle von Leitern und Halbleitern eine Gleichspannung und im Falle von Isolatoren eine hochfrequente Wechselspannung angelegt. Dadurch entstehende, in Richtung Ionenoptik 23 fliegende Neutralteilchen werden durch das Plasma 5 ionisiert und im angeschlossenen Massenspektrometer 22 auf ihre Masse hin untersucht.

Soll die Probe unmittelbar danach nach dem SBM/SNMS-Verfahren untersucht werden, dann ist zunächst erforderlich, die Potentialdifferenz zwischen Probe 14 und Plasma 5 derart zu reduzieren, dass Ionen des Plasmas die Probe entweder nicht mehr erreichen oder nur mit einer derart geringen Energie auftreffen, dass neutrale Teilchen oder Sekundärionen nicht mehr emittiert werden. Desweiteren ist die Ionenkanone 28 in Betrieb zu nehmen. Mittels darin erzeugter Ionen, z.B. 1 bis 5 keV-Ar-Ionen, wird die Probe beschossen. Dadurch werden Sekundärionen und Neutralteilchen aus der Probe ausgelöst. Die in Richtung Ionenoptik 23 fliegenden Neutralteilchen werden im Plasma 5 ionisiert und im Massenspektrometer analysiert. Um die unerwünschten Sekundärionen von den gewünschten, auf ihrem Weg zur Ionenoptik 23 durch das Plasma 5 ionisierten Neutralteilchen zu separieren, wird mit Hilfe der Ionenoptik 23 ein Energiefenster gesetzt. Diese Massnahme reicht zur Unterdrückung unerwünschter Sekundärionen aus, da diese im Vergleich zu den erwünschten Neutralteilchen in der Regel unterschiedliche Energieverteilungen haben.

Schliesslich kann die Probe 14 mit Hilfe der erfindungsgemässen Apparatur in der dargestellten Messposition nach dem SIMS-Verfahren untersucht werden. Dazu ist es – ausgehend vom SBM/SNMS-Verfahren – lediglich erforderlich, den Strom in der Windung 2 zu unterbrechen und damit das Plasma zu löschen. Zum Löschen des Plasmas kann auch die Zufuhr des Arbeitsgases unterbrochen werden. Durch den Beschuss mit Ionen aus der Ionenkanone 28 werden aus der Probe 14 Sekundärionen ausgelöst. In Richtung Ionenoptik 23 fliegende Ionen werden im nachfolgenden Spektrometer auf ihre Masse hin analysiert. Mit der Sekundärionen-Optik wird ein für die SIMS-Analyse geeignetes Energiefenster eingestellt.

Bei vorbekannten Anordnungen ist wegen der ungünstigeren Transmissionsverhältnisse eine Mikroanalyse mit Hilfe von Feinfokusionenquellen kaum möglich, da die erreichbaren absoluten Ionenströme bei derartigen Ionenquellen um einige Grössenordnungen niedriger sind als die bei bisherigen SNMS-Messungen benutzten grossflächigen Ionenkanonen. Beim dargestellten Ausführungsbeispiel hat die Probe sowohl für SNMS- als auch für SIMS-Messungen hinsichtlich der Transmission die günstigste Position, so dass der Einsatz von Feinfokusionenkanonen mit geringem Strom wesentlich bessere Ergebnisse liefert als bisher. Schliesslich können mit der Feinfokusionenquelle und dem Sekundärelektronen-Detektor 30 auch noch Sekundärelektronenbilder aufgenommen werden. Für diese Messvariante haben die einzelnen Bauteile ebenfalls eine günstige räumliche Zuordnung.

Im Vergleich zur vorbekannten Einrichtung entfällt bei der vorliegenden Einrichtung die Raumwinkelbegrenzung durch das Diaphragma, und die Öffnung der Ionenoptik wird durch die zerstäubten Teilchen besser ausgeleuchtet. Der dadurch erzielte Intensitätsgewinn für SIMS/SNMS beträgt etwa zwei Grössenordnungen.

Für alle möglichen Analysen wird nur ein Probentransfersystem benötigt.

Ein weiterer wesentlicher Vorteil ergibt sich bei der Untersuchung von Proben aus elektrisch isoliertem Material. Proben dieser Art haben bei der Analyse nach dem vorbekannten SBM/SNMS-Verfahren den Nachteil, dass sie sich durch den Ionenbeschuss aufladen. Um dieses zu verhindern, ist der zusätzliche Einsatz von Elektronenkanonen zur Ladungskompensation bekannt. Die Ladungskompensation kann auch durch Extraktion von Plasmaelektronen mit Hilfe des elektrischen Diaphragmas geschehen. Beim Arbeiten mit der erfindungsgemässen Apparatur ist eine solche Massnahme nicht erforderlich, da sich die Probe im Plasma befindet und ihre Aufladung durch die im Plasma vorhandenen Elektronen verhindert wird.

Mit der beschriebenen Apparatur können die unterschiedlichsten Analysen und Abtragbedingungen kombiniert oder nacheinander angewandt werden, z.B.

– hohe Abtragrate durch DBM (bei leitenden Proben durch Anlegen eines Probenpotentials, bei Isolatoren z.B. durch RF-Sputtern), gefolgt von einer statischen SIMS- oder SNMS-Analyse im DBM;

– hohe Abtragrate durch DBM, gefolgt von einer Mikroanalyse des Beschusskraters.

Diese beiden Beispiele zeigen, dass die in der Erfindungsmeldung vorgeschlagene apparative Konfiguration im Vergleich zu bisher bekannten SNMS-Anordnungen völlig neuartige analytische Experimente zulässt, zumal auch jederzeit ohne Umbauten die Aufnahme von Sekundärelektronenbildern möglich ist. Dazu wird der Elektronen-Detektor 30 in seine Betriebsstellung gebracht und eingeschaltet. Mit Ionen aus der Ionenquelle 28 – sei sie als Feinfokusionenquelle ausgebildet oder nicht – wird die Probe 14 rasterförmig abgetastet. Dadurch entstehende Sekundärelektronen werden vom Detektor 30 registriert und liefern ein Bild der Probenoberfläche.

## Patentansprüche

1. Einrichtung zur Durchführung des Sputtered Neutral Mass Spectrometry, (SNMS)-Verfahrens mit einer separaten Ionenquelle, einem Probenträger, einer Vorrichtung zur Erzeugung eines HF-Plasmas und einem Massenanalysator, dadurch gekennzeichnet, dass Ionenquelle (28) und Massenanalysator (22) nebeneinander auf derselben Seite des für das Hochfrequenzplasma (5) vorgesehenen Raumes (4) angeordnet sind und dass sich die auf dem Probenträger (31) gehalterte Probe (14) innerhalb des für das Hochfrequenzplasma (5) vorgesehenen Raumes (4) befindet.

2. Einrichtung nach Anspruch 1, dadurch gekennzeichnet, dass dem Massenanalysator (22) eine Ionenoptik (23) zur Einstellung eines Energiefensters vorgelagert ist.

3. Einrichtung nach Anspruch 1 oder 2, dadurch gekennzeichnet, dass neben der Ionenquelle (28) und dem Massenanalysator (22) ein Sekundärelektronen-Detektor (30) angeordnet ist.

4. Einrichtung nach Anspruch 1, 2 oder 3, dadurch gekennzeichnet, dass als Probenträger ein verschiebbarer Probenstab (13) vorgesehen ist, der auf der dem Massenanalysator (22) gegenüberliegenden Seite des für das HF-Plasma (5) vorgesehenen Raumes (4) angeordnet ist.

5. Einrichtung nach einem der vorhergehenden Ansprüche, dadurch gekennzeichnet, dass der für das HF-Plasma (5) vorgesehene Raum (4) von einem HF-durchlässigen, hochvakuumdichten Gefäss (1) gebildet wird, das von einer vorzugsweise einwindigen HF-Spule (2) umgeben ist.

6. Einrichtung nach Anspruch 4 und 5, dadurch gekennzeichnet, dass das Plasmagefäss (1) zylindrisch ausgebildet und mit zwei stirnseitig angeordneten Flanschen (6, 7) ausgerüstet ist, an denen auf der einen Seite der Massenanalysator (22) und die Ionenquelle (28) befestigt sind.

7. Einrichtung nach einem der vorhergehenden Ansprüche, dadurch gekennzeichnet, dass der Massenanalysator (22) ein Quadrupol-Massenfilter umfasst.

8. Einrichtung nach einem der vorhergehenden Ansprüche, dadurch gekennzeichnet, das die Ionenquelle (28) als Feinfokusionenquelle zur Durchführung von Mikroanalysen ausgebildet ist.

9. Einrichtung nach einem der vorhergehenden Ansprüche, dadurch gekennzeichnet, das die Probe (14) kippbar auf ihrem Probenträger (31) gehaltert ist.

10. Verwendung einer Einrichtung nach einem der vorhergehenden Ansprüche zur Durchführung des Secondary Ion Mass Spectrometry (SIMS)-Verfahrens.

11. Verwendung einer Einrichtung nach Anspruch 3 oder nach den Ansprüchen 3 und 8 zur Aufnahme von Sekundärelektronenbildern der Probe (14).

## Revendications

1. Appareil pour la mise en œuvre de la méthode de spectroscopie de masse de particules neutres pulvérisées, appelée méthode SNMS pour «Sputtered Neutral Mass Spectrometry», comprenant une source d'ions séparée, un porte-échantillon, un dispositif pour engendrer un plasma à haute fréquence et un analyseur de masse, caractérisé en ce que la source d'ions (28) et l'analyseur de masse (22) sont juxtaposés du même côté de l'espace (4) prévu pour le plasma à haute fréquence (5) et que l'échantillon (14), maintenu sur le porte-échantillon (31), est situé à l'intérieur de l'espace (4) prévu pour le plasma à haute fréquence (5).

2. Appareil selon la revendication 1, caractérisé en ce que l'analyseur de masse (22) est précédé d'une optique ionique (23) pour l'établissement d'une fenêtre énergétique.

3. Appareil selon la revendication 1 ou 2, caractérisé en ce qu'un détecteur d'électrons secondaires (30) est disposé à côté de la source d'ions (28) et de l'analyseur de masse (22).

4. Appareil selon la revendication 1, 2 ou 3, caractérisé en ce que le porte-échantillon est constitué par un barreau à échantillon (14) qui est disposé déplaçable du côté de l'espace (4) prévu pour le plasma HF (5) se trouvant à l'opposé de l'analyseur de masse (22).

5. Appareil selon l'une des revendications précédentes, caractérisé en ce que l'espace (4) prévu pour le plasma (5) est délimité par un récipient (1) qui est étanche au vide poussé et laisse passer la haute fréquence et qui est entouré par une bobine HF (2), de préférence à une spire.

6. Appareil selon les revendications 4 et 5, caractérisé en ce que le récipient à plasma (1) est cylindrique et est muni de deux brides (6, 7) disposées aux extrémités et auxquelles sont fixés, d'un côté, le système de transfert d'échantillon (8) et, de l'autre côté, l'analyseur de masse (22) et la source d'ions (28).

7. Appareil selon l'une des revendications précédentes, ⁴caractérisé en ce que l'analyseur de masse (22) comprend un spectromètre de masse quadripolaire.

8. Appareil selon l'une des revendications précédentes, caractérisé en ce que la source d'ions (28) est réalisée comme une source d'ions à focalisation fine pour l'exécution de microanalyses.

9. Appareil selon l'une des revendications précédentes, caractérisé en ce que l'échantillon (14) est maintenu basculant sur son porte-échantillon (31).

10. Utilisation d'un appareil selon l'une des revendications précédentes pour la mise en œuvre de la méthode de spectroscopie de masse de particules neutres pulvérisées, appelée méthode SNMS pour «Secondary Ion Mass Spectrometry».

11. Utilisation d'un appareil selon la revendication 3 ou selon les revendications 3 et 8 pour la prise d'images d'électrons secondaires de l'échantillon (14).

## Claims

1. Apparatus for carrying out the Sputtered Neutral Mass Spectrometry (SNMS) process with a separate ion source, a sample holder, a device for producing HF plasma and a mass analyser, characterised in that the ion source (28) and mass analyser (22) are arranged next to one another on the same side of the chamber (4) provided for the high frequency plasma (5) and in that the sample (14) secured on the sample holder (31) is located inside the chamber (4) provided for the high frequency plasma (5).

2. Apparatus according to claim 1, characterised in that an ion lens system (23) is provided in front of the mass analyser (22) for adjusting an energy window.

3. Apparatus according to claim 1 or 2, characterised in that a secondary electron detector (30) is arranged next to the ion source (28) and the mass analyser (22).

4. Apparatus according to claim 1, 2 or 3, characterised in that a movable sample rod (13) which is arranged on the side of the chamber (4) provided for the HF plasma (5) opposite the mass analyser (22) is provided as sample holder.

5. Apparatus according to one of the preceding claims, characterised in that the chamber (4) provided for the HF plasma (5) is formed by an HF-permeable container (1) which is tight to high vacuum and is surrounded by a preferably single-turn HF coil (2).

6. Apparatus according to claims 4 and 5, characterised in that the plasma container (1) is cylindrical in construction and is equipped with two flanges (6, 7) which are arranged on the end faces and on one side of which the sample transfer system (8) is fixed and on the other side of which the mass analyser (22) and the ion source (28) are fixed.

7. Apparatus according to one of the preceding claims, characterised in that the mass analyser (22) comprises a quadrupole mass filter.

8. Apparatus according to one of the preceding claims, characterised in that the ion source (28) is designed as a fine focus ion source for carrying out microanalysis.

9. Apparatus according to one of the preceding claims, characterised in that the sample (14) is held in tilting fashion on its sample holder (31).

10. Use of apparatus according to one of the preceding claims for carrying out the Secondary Ion Mass Spectrometry (SIMS) process.

11. Use of apparatus according to claim 3 or according to claims 3 and 8 for receiving secondary electron formers of the sample (14).

-1 / 1-

FIG. 1

FIG. 3

FIG. 2